# EUROPEAN PATENT APPLICATION

(11) **EP 3 485 918 A1**
(43) Date of publication of application: **22.05.2019**
(21) Application number: 17202812.8
(22) Date of filing: 21.11.2017
(51) Int. Cl.: A61L 27/20, A61L 27/38, A61L 27/50, A61L 27/52, A61L 27/58, C08L 5/08, C08L 5/12

(54) **GEL COMPOSITION FOR 3D PRINTING OF CELL SUSPENSIONS**

(71) Applicant: ETH Zürich, 8092 Zürich (CH)
(72) Inventor: The designation of the inventor has not yet been filed
(74) Representative: Schulz Junghans Patentanwälte PartGmbB

(57) **Abstract**

The invention relates to a method for providing a three-dimensional structure comprising viable cells. A volume of an aqueous composition comprising a glycosaminoglycan component, a polysaccharide component, fumed silica, growth medium and live cells is subjected to a shear force, whereby it is liquefied, and applied to a three-dimensional structure. The total mass of the glycosaminoglycan, the polysaccharide and the fumed silica, in relation to the total mass of the aqueous composition, ranges from 3% to 9% (w/w). The invention further relates to a composition for use in the method of the invention, and to gel objects made by the method of the invention.

## Description

The invention relates to aqueous gel compositions comprising a glycosaminoglycans, a gel forming polysaccharide, a thixotropic agent (particularly fumed silica or a smectic clay) and live cells. The invention further relates to a method of assembling three-dimensional objects from such aqueous gel compositions by additive manufacturing (3D printing).

### Introduction

Bacteria may create physical matter in the form of biofilms that allow them to survive even in hostile environments. Biofilms adapt their mechanical properties under stress to match conditions imposed by the surrounding environment with a great diversity of biopolymers. During growth in biofilms, bacteria can also form and degrade many compounds, which are often used to synthesize industrially relevant chemical compounds, biopolymers, enzymes and proteins. Moreover, bacteria are able to form calcium carbonate, magnetites, and biopolymers, which could lead to the formation of a new generation of biomineralized composites, biodegradable plastics, and functional materials for biomedical applications. Despite these remarkable features, the use of the programmable biochemical machinery of bacteria to create "living materials" with controlled 3D shape, microstructure and dynamic metabolic response remains largely unexplored. This is due to the lack of manufacturing tools that enable immobilization of bacteria in a biocompatible medium that can be further processed into functional materials with well-defined 3D geometry and site-specific cellular and chemical composition.

Strategies to immobilize bacteria while maintaining their metabolic activity are known in the art, including adsorption on surfaces, cell crosslinking and encapsulation. In contrast to immobilization on hard surfaces, embedding bacteria in hydrogels provides an ideal living environment with a high water content while allowing nutrients to flow in and waste products to diffuse out of the hydrogel.

Bacteria such as *Acetobacter xylinum,* also referred to as *Gluconoacetobacter xylinus,* are able to secrete nano-cellulose hydrogels directly at the water-air interface with significant mechanical strength. This biofilm formation process can be used for the production of artificial skin, medical patches and in food products. In particular, bacterial cellulose has been found to be extremely useful in the medical industry due to their cell biocompatibility, making it ideal for tissue engineering. So far, bacterial cellulose has been manufactured *in situ* in the form of surface-patterned implants, ear transplants, and potential blood vessels by diffusing oxygen through nano-patterned and 3D shaped silicone molds. In these examples, biofilms have been mainly formed as a film in a non-immobilized state at a variety of surfaces and interfaces by depositing on the desired substrate a layer of bacteria initially suspending in a fluid culture medium. Immobilizing bacteria in a viscoelastic matrix that could be free-formed into intricate geometries and combined with different microorganisms to achieve site-specific cellular and chemical composition control would enable the fabrication of living materials with thus far inaccessible dynamic functionalities.

Overcoming the geometrical constraints and the lack of site-specific control of current biofilm growth approaches will lead to novel design possibilities in microbial biosensors and provide mechanical stability to entrapped cells in biotechnological applications.

The present invention provides improved means and methods for using matrix-associated live cell containing composites in technology. This objective is attained by the subject matter of the independent claims, with further advantageous embodiments set forth in the dependent claims.

### Terms and definitions

The term *glycosaminoglycan* in the context of the present specification relates to long unbranched polysaccharides of a repeating disaccharide unit comprising at least one amino sugar in the disaccharide unit. The repeating unit (except for keratan) consists of N-acetylglucosamine or N-acetylgalactosamine along with glucuronic acid or iduronic acid or galactose. The term *glycosaminoglycan* in the context of the present specification particularly includes hyaluronic acid, hyaluronan sulfate, heparin, heparin sulfate, chondroitin sulfate and dermatan sulfate. The term *glycosaminoglycan* in the context of the present specification in particular relates to a compound, or a mix of compounds, selected from hyaluronic acid and hyaluronan sulfate.

The term *gel forming polysaccharide* in the context of the present specification relates to a polysaccharide capable of forming an elastic hydrogel in aqueous phases, in particular a polysaccharide capable of forming a hydrogel elastic in response to a stress ranging from 0 to 5 kPa in aqueous phases. More particularly, the term *gel forming polysaccharide* in the context of the present specification relates to a compound, or a mix of compounds, selected from alginate, alginate sulfate, gellan sulfate, starch, modified starch, gelatin, pectin, cellulose, bacterial cellulose, chitosan , gellan gum, acylated and/or sulfated gellan gum, guar gum, cassia gum, konjac gum, Arabic gum, ghatti gum, locust bean gum, xanthan gum, xanthan gum sulfate, carrageen, carrageen sulfate, and a mixture of any of the gel forming polysaccharides mentioned previously.

The term *fumed* or *pyrogenic silica* in the context of the present specification relates to a composition essentially composed of amorphous silica nanoparticles (primary particle size 5-50 nm) fused into branched, three-dimensional secondary particles, which agglomerate into tertiary particles at concentrations higher than 1 %.

The term *smectic clay* in the context of the present specification relates to montmorillonite, beidellite, nontronite, saponite, laponite, hectorite and similar.

The term growth medium in the context of the present specification relates to any mixture of ingredients necessary to provide nutrient, pH stability and osmotic concentration for the cells contained in the composition to be viable.

### Description of the invention

The present invention relates to a 3D printing platform that, besides 3D shaping, enables the digital fabrication of bacteria-loaded hydrogel materials with full control over the spatial distribution and concentration of living species in complex 3D architectures. The inventors developed what could be referred to as a functional living ink, in some instances referred to as 'Flink' herein, that is a biocompatible immobilization medium that exhibits the viscoelastic properties required for 3D printing through multi-material direct ink writing. The freedom of shape and material composition provided by this printing technique is combined with the metabolic response of living matter to enable the digital fabrication of bacteria-derived living materials featuring unprecedented functionalities such as adaptive behaviour, pollutant degradation and structure formation in the form of cellulose reinforcement. As a novel additive manufacturing approach, Flink 3D printing, opens the possibility to combine different organisms and chemistries in a single process, allowing for the digital shaping of living materials into new geometries and adaptive functional architectures.

In a most general sense, the invention relates to compositions that comprise live cells (particularly, but not limited to, prokaryotic cells) in an aqueous growth medium suited to sustain viability of the cells. The composition of the invention further comprises three components that combine in their properties to enable 3D printing of the composition.

The first component is a viscous hydrogel, particularly comprising or essentially consisting of a glycosaminoglycan component. The second component is an elastic hydrogel component, particularly comprising or essentially consisting of polysaccharides capable of forming an elastic hydrogel.

The third component is a thixotropic agent that imparts viscoelastic properties on the composition, particularly a yield stress above 100 Pa.

A first aspect of the invention relates to a method for providing a three-dimensional structure comprising viable prokaryotic or eukaryotic cells. This method comprises the steps of
a) providing an aqueous composition comprising
   i) a glycosaminoglycan component,
   ii) a gel forming polysaccharide component,
   iii) a thixotropic component,
   iv) a growth medium component,
   v) a population of live bacterial or eukaryotic cells.
b) repeatedly or continuously exerting a shear force on a volume of said aqueous composition, thereby liquefying said volume, and
c) applying said volume of said aqueous composition to form a three-dimensional structure.

The sum of the mass of each the glycosaminoglycan component, the gel forming polysaccharide component and the thixotropic component, in relation to the total mass of the aqueous composition, ranges from 3% to 9% (w/w), particularly from 4,5% to 6%, 7% or 8% (w/w).

The glycosaminoglycan component consists of a gel forming glycosaminoglycan. In certain embodiments, the glycosaminoglycan is selected from the group of hyaluronic acid, chondroitin sulfate, keratan sulfate, heparin, dermatan sulfate and heparan sulfate.

In certain embodiments, the glycosaminoglycan component essentially consists of hyaluronic acid.

The polysaccharide component consists of a gel forming polysaccharide, such as, by way of non-limiting example, alginate, alginate sulfate, gellan sulfate, starch, modified starch, gelatin, pectin, cellulose, bacterial cellulose, chitosan, gellan gum, acylated and/or sulfated gellan gum, guar gum, cassia gum, konjac gum, Arabic gum, ghatti gum, locust bean gum, xanthan gum, xanthan gum sulfate, carrageen, carrageen sulfate, or a mixture of any of the above gel forming polysaccharides. In certain embodiments, the gel forming polysaccharide component is a carageenan, particularly a kappa (k-) carageenan, or agar.

While in the broadest chemical sense, a glycosaminoglycan can also be summed under polysaccharides, the two components are mentioned separately because they fulfil different roles within the composition. The accuracy of printing, i.e. the tendency to form and maintain a precise shape after being ejected through the printer nozzle, is significantly higher if both the glycosaminoglycan and a distinct gel-forming polysaccharide are present.

The printing accuracy is the sum of different variables, particularly the elasticity and the yield stress. Combining the glycosaminoglycan component with the gel forming polysaccharide results in a synergistic effect shown by the higher viscosity compared to the sum of the viscosities of each constituent alone at the same individual concentrations. Simplified we can state that a higher viscosity in combination with a high yield stress does lead to an improved printing accuracy.

For printing, a gel only characterized by viscous behaviour is not useful since it would trip out of the nozzle (low viscosity) or simply not flow through it (high viscosity). An additional elastic component is required, which is contributed by the gel forming polysaccharide component, particularly by the k-carageenan, which further provides nutrients in the form of polysaccharides to the cells. The glycosaminoglycan component on the other hand provides the viscous nature as well as good cell compatibility. The thixotropic agent is an inert, non-toxic component that by itself forms a viscoelastic gel, further improving/supporting the viscous as well as the elastic characteristics of the gel formed by the glycosaminoglycan + polysaccharide components.

One important feature of hyaluronic acid is its physiological interaction with certain receptors on a number of cell types, which adds to their viability in the mixture. Of note, hyaluronic acid interacts with the cell-surface receptor cluster determinant 44 (CD44) and the receptor for hyaluronate-mediated motility (RHAMM). CD44 plays a key role in tissue organization by mediating ECM remodelling, cell-cell interactions and cell-matrix interactions. A normal wound healing model in CD44-knockout mice exhibited decreased cell migration, motility and ECM turnover. Interactions between HA and RHAMM can result in a variety of downstream signalling pathways that affect protein kinases such as focal adhesion kinases, MAP kinases, phosphatidylinositol kinases, and tyrosine kinases (see: Lam et al., Acta Biomater, 10, 2014)

In certain embodiments, the glycosaminoglycan component, particularly hyaluronic acid, used in the composition of the invention is selected from a molecular mass range of 10³-10⁴ kDA. The thermal treatment is expected to reduce the average molecular mass of this component to less than 10³ kDa, at which range the viability of mammalian cells, but not prokaryotic cells, may be compromised. This glycosaminoglycan component forms a predominantly viscous hydrogel (on its own), whereas the gel-forming polysaccharide forms a predominantly elastic hydrogel (on its own).

In certain embodiments, the gel forming polysaccharide component is selected from a molecular mass range of 10²-10³ kDa, particularly for k-carageenan.

In embodiments in which the gel-forming polysaccharide component is k-carrageenan, the aqueous composition additionally comprises potassium ions.

### Thixotropic agent component

The thixotropic agent is added to the viscous and/or elastic gel component in order to make the composition viscoelastic. In certain embodiments, the thixotropic agent is fumed silica. In certain embodiments, the thixotropic agent is a smectic clay mineral, particularly selected from the group of hydrous aluminium phyllosilicates having variable amounts of cations (iron, magnesium, (earth)-alkali metals) characterized by two-dimensional sheets of corner sharing SiO₄ tetrahedra and/or AlO₄ octahedra with the sheet chemical composition of (Al,Si)₃O₄. Non limiting examples are kaolinite, serpentine for 1:1 clays and laponite, talk, vermiculite and montmorillonite for 2:1 clays.

Addition of the thixotropic agent leads to compositions characterized by a well-defined yield stress above 100 Pa.

In certain embodiments, the thixotropic agent component essentially consists of, or comprises, fumed (pyrogenic) silica.

In certain embodiments, the volume of the composition that is applied by 3D printing ranges between 0,05 µl and 1 µl per 1 mm print path.

### Compositions

In certain embodiments, the mass of the glycosaminoglycan component ranges from 1% to 2%, the mass of the k-carrageenan component ranges from 1% to 2% and the mass of the thixotropic component ranges from 1% to 2%, in relation to the total mass of the aqueous composition, respectively.

In certain embodiments, each of the mass of the glycosaminoglycan component, the mass of the gel-forming polysaccharide component and the mass of the thixotropic component, in relation to the total mass of the aqueous composition, are individually and independently from each other selected to range from 1,0 % to 5,0% (w/w), particularly from 1 % to 3%.

In certain embodiments, the mass of the glycosaminoglycan component, the mass of the gel-forming polysaccharide component and the mass of the thixotropic component are selected so that the component having the highest mass among said components is equal or less (≤) than 1,5 times, particularly 1,2 times, the lowest mass among said components.

In certain embodiments, the sum of the mass of the glycosaminoglycan component and the mass of the gel-forming polysaccharide component is selected to range between 1 1/3 times and 3 times the mass of the thixotropic component.

In certain embodiments, the mass of the glycosaminoglycan component G, the mass of the gel-forming polysaccharide component P and the mass of the thixotropic component FS are selected from Table 1 (all values in mass/mass %):

| No | G | P | FS | total |
|---|---|---|---|---|
| Compositions used for cell cultivation, not 3D printing: | | | | |
| 1 | 0 -1% | 1% | 1% | 2-3% |
| 2 | 1% | 0 -1% | 1% | 2-3% |
| 3 | 1% | 1% | 0-1% | 2-3% |
| 4 | 1% | 1% | 1% | 3% |

| Compositions used for 3D printing | | | | |
|---|---|---|---|---|
| 5 | 1 - 1.5% | 1 - 1.5% | 1 - 1,5% | 3,5 - 4,5% |
| 6 | 1% | 1% | 1-3% | 3,5-5% |
| 7 | 1% | 1-3% | 1% | 3-5% |
| 8 | 1-3% | 1% | 1% | 3-5% |
| 9 | 1-3% | 1-3% | 1% | 3-7% |
| 10 | 1% | 1-3% | 1-3% | 3-7% |
| 11 | 1-3% | 1% | 1-3% | 3-7% |
| 12 | 2% | 2% | 2% | 6,0 % |
| 13 | 2-3% | 2-3% | 2-3% | 6,0 - 9,0% |
| 14 | 3% | 3% | 3% | 9,0 % |

The inventors have found that compositions having a content of G or P below 1%, or a sum G and p below 2%, do not work well as they do not keep the 3D printed shape. Combinations of each component at above 1 % work well, with optimal results being attained at above 4% total content of G+P+FS. The inventors have found that compositions having a total of G+P+FS >9% are too thick to print.

The inventors have also found that leaving one component out, combining 1% + 1% of either G, P, and FS in the combinations (G-P-FS) 0-1-1, 1-1-0, 1-0-1 does not lead to a working ink.

In certain embodiments, the shear force applied in step b) is 100 Pa or greater. The inventors found that a minimal shear force of 100 Pa is necessary to liquefy the ink. The ink only liquefies in the nozzle where high shear forces are present.

In certain embodiments, the glycosaminoglycan component comprises photo-polymerizable carbon-carbon double bonds, particularly wherein said glycosaminoglycan component comprises methacrylic acid residues. The methacrylate content is selected in a range between 1 and 50%, particularly from 2% to 40%. It can be produced as published by Leach et al. in Biomaterials 26 (2005): 125-135. Other chemistries than the crosslinking by radical polymerization with GMA are feasible also. If light is intended to start the process of crosslinking the glycosaminoglycan component, methacrylate is of particular utility. Other crosslinking possibilities include crosslinking by aldehyde (especially glutaraldehyde) or azide (adipic acid dihydrazide), crosslinking by addition reaction with isocyanate as well as crosslinking by condensation reaction with imides and amines.

In certain embodiments, the three-dimensional structure is exposed to light suitable for polymerization of said photo-polymerizable carbon-carbon double bonds. In certain embodiments, the wavelength is selected from the visible and near ultraviolet region. An example of an advantageous wavelength promoting bacteria survival is 365 nm, for example during 60s, at 90 mW.

In certain embodiments, the population of live cells comprises fungal cells. In certain embodiments, the population of live cells comprises plant cells.

In certain embodiments, the population of live cells comprises live bacterial cells. In certain embodiments, the population of live bacterial cells comprises, or essentially consists of, cells that deposit a solid matrix, particularly cellulose or calcium carbonate. In certain of these embodiments, the cells are selected from Acetobacter xylinum, Gluconacetobacter hansenii, Gluconacetobacter xylinus, Acetobacter sp., Sporosarcina pasteurii, Deleya halophile and Proteus mirabilis, particularly selected from A. xylinum strains BRC5, BPR2001, E25 or NUST4.1, G. hansenii strain PJK, Acetobacter sp. strains V6 or A9, G. xylinus strains K3, IFO 13773 and RKY5, Sporosarcina pasteurii ATCC 11859. In certain embodiments, the cells are selected from *Rhizobium, Alcaligenes, Acetobacter, Azotobacter, Pseudomonas, Salmonella* and *Sarcina ventriculi.*

In certain embodiments, the population of live cells comprises live bacterial cells selected from the group comprising *Magnetospirillum gryphiswaldense* MSR-1, *Magnetospirillum magneticum* AMB-1, *Magnetospirillum magneticum* MGT-1, *Magnetovibrio* MV-1, *Magnetococcus* sp. MC-1, *Magnetospirillum* sp. WM-1 *Magnetospirillum magnetotacticum* MS-1 and *Desulfovibrio magneticus* RS-1.

Another aspect of the invention relates to an aqueous composition for use in the method of the invention. This composition comprises
i) a glycosaminoglycan component, particularly a hyaluronic acid component,
ii) a gel forming polysaccharide component,
iii) a thixotropic agent component, particularly a fumed (pyrogenic) silica component,
iv) a growth medium component,
v) a population of live bacterial cells,

The sum of the mass of each the glycosaminoglycan component, the polysaccharide component and the thixotropic component, in relation to the total mass of the aqueous composition, ranges from 3% to 9% (w/w).

The embodiments mentioned above that relate to the glycosaminoglycan, polysaccharide and thixotropic components as regard specific compounds and mass ratios apply to this aspect of the invention also.

Yet another aspect relates to a solid gel volume having a discrete three-dimensional form. This solid gel comprises
i) a glycosaminoglycan component, particularly a hyaluronic acid component,
ii) a gel forming polysaccharide component, particularly a k-carageenan component,
iii) a thixotropic agent component, particularly a fumed (pyrogenic) silica component,
iv) a population of live bacterial cells,
v) optionally, a growth medium component,
wherein said solid gel is characterized by
- an elastic modulus of 0.1-10kPa and/or
- a dynamic yield stress of 10-500 Pa and/ or
- an instant recovery (less than a second) of the viscoelastic network.

In certain embodiments, the mass of the glycosaminoglycan component, the mass of the gel-forming polysaccharide component and the mass of the thixotropic component are selected from Table 1.

In certain embodiments, the glycosaminoglycan component comprises photopolymerizable carbon-carbon double bonds, particularly wherein said glycosaminoglycan comprises crosslinked methacrylic acid residues.

Another aspect of the invention relates to the use of a solid gel volume according to the invention, wherein the bacterial cells are P. putida cells, in a method for degrading phenols. The invention further encompasses the following items:
1. A method for providing a three-dimensional structure comprising viable cells, comprising the steps of
   a) providing an aqueous composition comprising
      i) a glycosaminoglycan component, particularly a hyaluronic acid component,
      ii) a gel forming polysaccharide component,
      iii) a thixotropic agent component,
      iv) a growth medium component, and
      v) a population of live [bacterial or eukaryotic] cells,
      wherein the sum of the mass of each the glycosaminoglycan component, the gel forming polysaccharide component and the fumed silica component, in relation to the total mass of the aqueous composition, ranges from 3% to 9% (w/w).
   b) repeatedly exerting a shear force on a volume of said aqueous composition, and
   c) applying said volume of said aqueous composition to a three-dimensional structure.
2. The method according to item 1, wherein said gel forming polysaccharide component is a carageenan, particularly a kappa (k-) carageenan, or agar.
3. The method according to item 1 or 2, wherein the thixotropic agent component is selected from the group comprising fumed (pyrogenic) silica and smectic clay minerals.
4. The method according to any one of the preceding items, wherein said volume of the aqueous composition applied per step ranges from 0,05 µl to 1 µl per 1 mm print path.
5. The method according to any one of the preceding items, wherein the sum of the mass of each the glycosaminoglycan component, the gel forming polysaccharide component and the fumed silica component, in relation to the total mass of the aqueous composition, ranges from 4,5% to 9%, particularly from 4,5% to 8%, more particularly from 4,5% to 7% or 4,5% to 6% (all % as w/w).
6. The method according to any one of the preceding items, wherein each of the mass of the glycosaminoglycan component, the mass of the gel-forming polysaccharide component and the mass of the thixotropic agent component, in relation to the total mass of the aqueous composition, are individually and independently from each other selected to range from 1,0 % to 5,0%, particularly from 1% to 3%, more particularly from 1,2% to 2,5% or from 1,3% to 2,5%, even more particularly from 1,5% to 2,5% (all percentages given as w/w).
7. The method according to any one of the preceding items, wherein the mass of the glycosaminoglycan component, the mass of the gel-forming polysaccharide component and the mass of the thixotropic agent component are selected so that the component having the highest mass among said components is equal or less (≤) than 1,5 times, particularly ≤ 1,2 times, the lowest mass among said components.
8. The method according to any one of the preceding items, wherein the sum of the mass of the glycosaminoglycan component and the mass of the gel-forming polysaccharide component is selected to range between 1 1/3 times and 3 times the mass of the thixotropic agent component.
9. The method according to any one of the preceding items, wherein the mass of the glycosaminoglycan component, the mass of the gel-forming polysaccharide component and the mass of the thixotropic agent component are selected from Table 1.
10. The method according to any one of the preceding items, wherein the shear stress applied in step b) is 100 Pa or greater.
11. The method according to any one of the preceding items, wherein said glycosaminoglycan component comprises photopolymerizable carbon-carbon double bonds, particularly wherein said glycosaminoglycan component comprises methacrylic acid residues.
12. The method according to item 11, wherein the three-dimensional structure is exposed to light suitable for polymerization of said photopolymerizable carbon-carbon double bonds.
13. The method according to any one of the preceding items, wherein said population of live bacterial cells comprises, or essentially consists of, cells that deposit a solid matrix.
14. The method according to item 14, wherein said solid matrix is selected from cellulose, calcium carbonate and magnetite.
15. The method according to item 13 or 14, wherein said cells are selected from
   a. Acetinobacter xylinum, Gluconacetobacter hansenii, Gluconacetobacter xylinus, Acetobacter sp., Sporosarcina pasteurii, Deleya halophile and Proteus mirabilis, particularly selected from the group comprising A. xylinum strains BRC5, BPR2001, E25 or NUST4.1, G. hansenii strain PJK, Acetobacter sp. strains V6 or A9 , G. xylinus strains K3, IFO 13773 and RKY5, Sporosarcina pasteurii ATCC 11859, or from
   b. the group comprising Rhizobium, Alcaligenes, Azotobacter, Pseudomonas, Salmonella, Sarcina ventriculi and / or
   c. magnetotactic bacteria, particularly selected from the group comprising *Magnetospirillum gryphiswaldense* MSR-1, *Magnetospirillum magneticum* AMB-1, *Magnetospirillum magneticum* MGT-1, *Magnetovibrio* MV-1, *Magnetococcus* sp. MC-1, *Magnetospirillum* sp. WM-1 *Magnetospirillum magnetotacticum* MS-1 and *Desulfovibrio magneticus* RS-1.
16. An aqueous composition comprising
   i) a glycosaminoglycan component, particularly a hyaluronic acid component,
   ii) a gel forming polysaccharide component,
   iii) a thixotropic agent component, particularly a fumed (pyrogenic) silica component,
   iv) a growth medium component, and
   v) a population of live bacterial cells,
   wherein the sum of the mass of each the glycosaminoglycan component, the k-carageenan component and the fumed silica component, in relation to the total mass of the aqueous composition, ranges from 3% to 9% (w/w).
17. A solid gel volume having a discrete three-dimensional form, said solid gel comprising
   i) a glycosaminoglycan component, particularly a hyaluronic acid component,
   ii) a gel forming polysaccharide component, particularly a k-carageenan component,
   iii) a thixotropic agent component, particularly a fumed (pyrogenic) silica component,
   iv) a population of live bacterial cells, and
   v) optionally, a growth medium component,
   wherein said solid gel is characterized by
   - an elastic modulus of 0.1-10 kPa and/or
   - a yield strain of 10-50% and/or
   - a dynamic yield stress of 10-500 Pa and/ or
   - an instant recovery (less than a second) of the viscoelastic network.
18. The solid gel volume according to item 17, wherein the mass of the glycosaminoglycan component, the mass of the gel-forming polysaccharide component and the mass of the thixotropic agent component are selected from Table 1.
19. The solid gel volume according to item 17 or 18, wherein the glycosaminoglycan component comprises photopolymerizable carbon-carbon double bonds, particularly wherein said glycosaminoglycan comprises crosslinked methacrylic acid residues.
20. Use of a solid gel volume according to item 17 to 19, wherein the bacterial cells are P. putida cells, in a method for degrading phenols.

Wherever alternatives for single separable features such as, for example, an isotype protein or glycosaminoglycan, polysaccharide or bacterial cell type are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

### Brief description of the figures

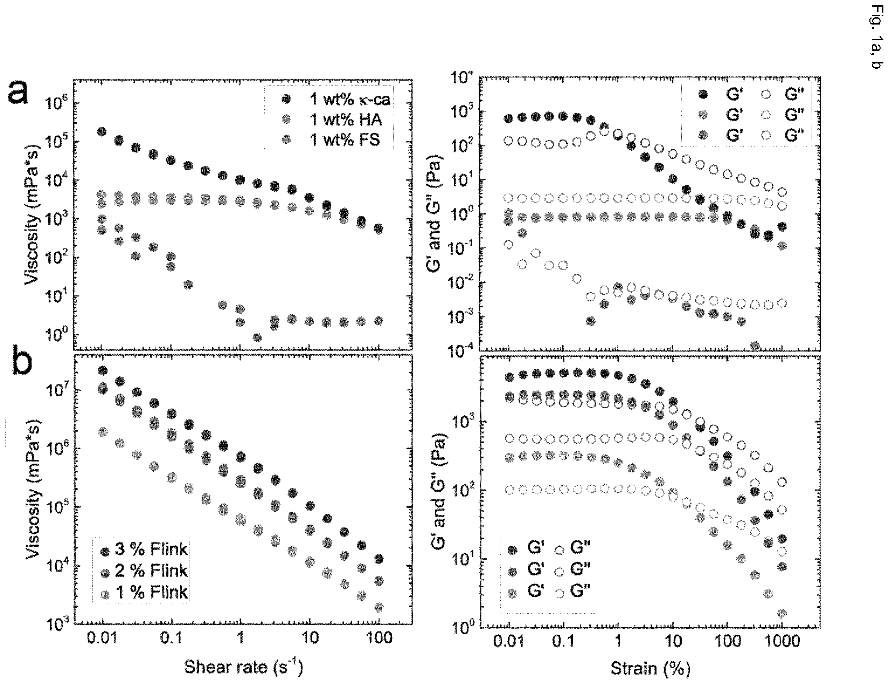

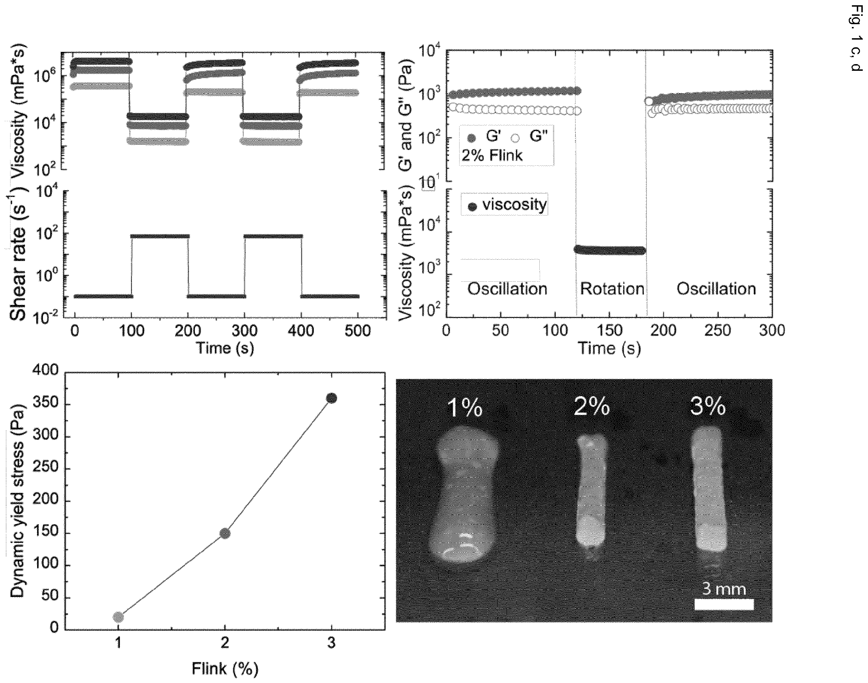

- Fig. 1: shows (a) the rheological properties of the individual components of an exemplary ink: 1 wt% k-carrageenan, 1 wt% hyaluronic acid and 1 wt% fumed silica in LB bacterial media and (b) in combination as functional living ink 'Flink' at concentrations of a total of components of 3%, 6% and 9% (wt%) respectively. The steady-state flow behavior of the inks is measured by viscosity curves at increasing and decreasing shear rates. The elastic (G') and viscous (G") moduli are measured by oscillatory amplitude sweeps (strain = 0.01-1000%, angular frequency = 1 rad/s). (c) To simulate printing conditions, alternating high
(70 s⁻¹) and low (0.1 s⁻¹) shear rates were applied to 3, 6, and 9% (total weight of solid components) Flink. Instantaneous recovery of the viscoelastic network of 1 wt% Flink is shown by a sudden shear process (70 s⁻¹) followed by an oscillatory time sweep. (d, left) Yield stresses of different Flink concentrations measured in stress-controlled measurements. (d, right) shows the effect of yield stress and elasticity of the Flink on structure retention in printed filaments.

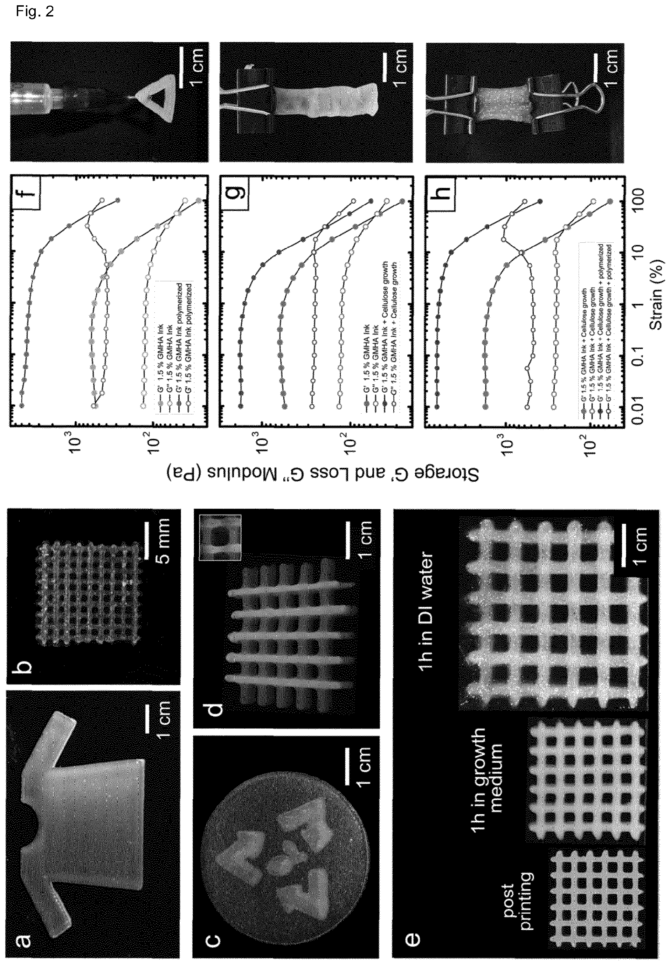

- Fig. 2: shows the 3D printing accuracy of a 4.5 wt% composition (1,5% glycosaminoglycan, 1,5% polysaccharide and 1,5% FS), swelling and mechanical properties after polymerization and bacterial growth. (a-c) Flink containing bacteria was 3D printed to obtain a variety of shapes at high precision. (d) Multi-material 3D printing allows localizing different bacteria in specific regions of the scaffold. *Pseudomonas putida* is colored with DAPI (blue) and is deposited to form horizontal lines, whereas *Bacillus subtilis* is colored with Nile Red (green) and printed to generate vertical lines. (e) 6 wt% (2%+2%+2%) Flink was modified with glycidyl methacrylate HA to form a UV-crosslinkable (365 nm, 60s, 90 mW) and therefore water-insoluble hydrogel. Swelling properties are shown right after printed printing, incubated for 1 hour in bacterial media and in DI water. The network properties (elastic G' and viscous G" moduli) of GMHA ink (4.5 wt%) before and after polymerization (f), after bacterial growth of *Acetobacter xylinum* (g) and after bacterial growth and polymerization (h) measured with amplitude sweeps (strain = 0.01-100%, angular frequency = 1 rad/s).

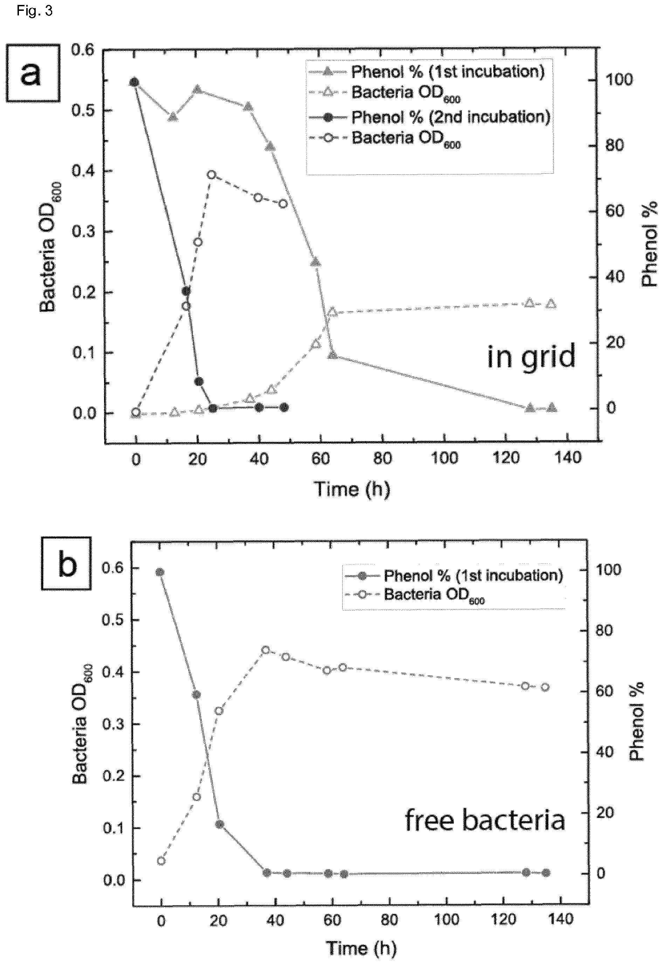

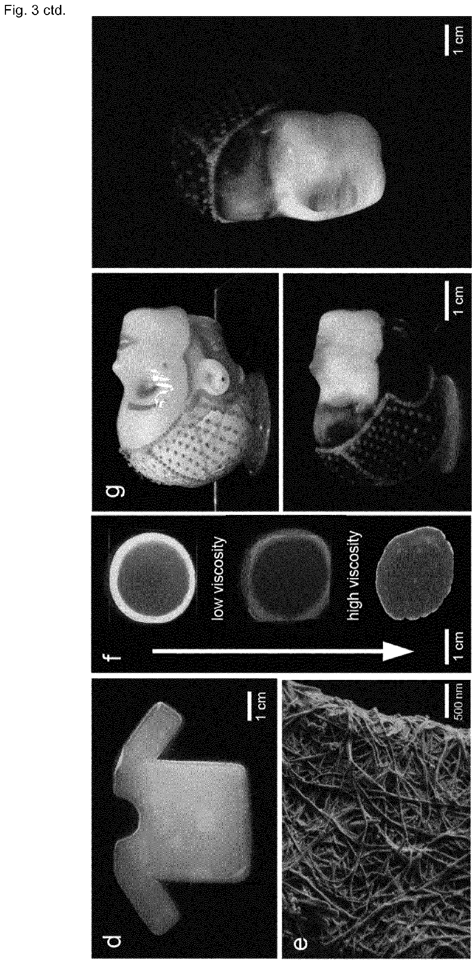

- Fig. 3: shows 3D printed bacteria-functionalized Flink with complex shapes for bioremediation and biomedical applications. (a) A Flink grid with Pseudomonas putida, a known phenol degrader, was printed and inoculated in a phenol-containing minimal salts medium with phenol as the only carbon source. Phenol degradation was plotted versus the optical density (OD600) of the bacteria as an indicator of growth. As a control, the same bacteria concentration was incubated as a free-floating culture. (b) The pre-incubated grid was inoculated for a second time in a phenol containing minimal salts media. This time, phenol degradation happened as fast as with the free-floating control. (c) The P. putida grid before (top) and after (bottom) inoculation visualized with ethidium bromide coloring of bacterial DNA (343nm). (d) The in-situ bacterial cellulose formation of A. xylinum forms a 3D printed scaffold in the shape of a T-shirt, visualized with a cellulose specific fluorescent dye. (e) The bacterial cellulose nano-fibril network under SEM. (f) Growth of bacterial cellulose depends on oxygen availability and the viscosity (total weight% of components: 3wt%, 4.5 wt% and 6 wt%) of the Flink. Cellulose is only present in regions of high oxygen and low to medium viscosities. (g) A doll face was scanned and a Flink containing A. xylinum was printed onto the face using a custom-built 3D printer. In-situ cellulose growth leads to the formation of a cellulose-reinforced hydrogels that, after removal of all biological residues, can serve as a skin replacement.

### Examples

To demonstrate the effect of the metabolic activity and growth of bacteria in 3D printed structures, two examples are presented displaying the magnitude of possibilities. As a first example, the phenol degradation capability of *P. putida* immobilized in a 3D printed structure is demonstrated for bioremediation applications. A second example shows the growth and in-situ bacterial cellulose formation of *A. xylinum* in a 3D printed structure relevant for biomedical applications. With the presented multi-material additive manufacturing process, versatile structures can be created by spatial control over the hydrogel composition and localization of different bacteria strains.

### Example 1: Viscoelastic and shear thinning bioink with structure recovery

The composition of Example 1 enables distortion free and accurate 3D printing of bacteria loaded hydrogels.

The rheological properties of the hydrogel were measured to characterize the single components and combined together in the hydrogel before, during and after 3D printing (see Fig. 1). To reach ideal rheological properties for Direct Ink Writing (DIW) and high survival of bacteria, three non-toxic components κ-carrageenan (κ-ca), hyaluronic acid (HA) and fumed silica (FS) were solubilized in lysogeny broth (LB) to form a hydrogel ink. Different solid-loading concentrations were tested for proliferation and mobility of the bacteria, namely 1 wt% Flink (each component being present at 3% w/w: κ-ca:HA:FS as 1+1+1), 6 wt% Flink (κ-ca:HA:FS as 2+2+2) and 9 wt% Flink (κ-ca:HA:FS as 3+3+3). An ink design with high water content (95.5 wt%) was identified as an advantageous embodiment for bacterial growth and mobility inside the gel. A bacteria toxicity assay for Gram positive (*B*. *subtilis*) and Gram negative (*P. putida*) bacteria revealed that all ink components are fully biocompatible and that post processing of the composition with UV exposure at 365 nm for 60s at 90mW as well as the presence of free radicals during polymerization is not hazardous to the bacteria (100% viability).

To determine the individual rheological properties of the hydrogel components, the viscosities of the single components (κ-ca, HA, FS) were measured with increasing and decreasing shear rates (Figure 1 a). The components show a weak shear thinning behavior, with an inconstant shear thinning viscosity with increasing shear rates. For κ-ca, HA, and FS no hysteresis was observed for decreasing and increasing shear rates. In oscillatory amplitude sweeps, solutions of FS at 1 wt% display only a weak viscosity at low strains, whereas κ-ca and HA form predominantly elastic and viscous networks, respectively. κ-ca and HA, both being natural hydrogel formers, were added to increase the water content and therefore create a bacteria-compatible environment, whereas FS was added as a viscosity modifier to increase the viscosity at low shear rates. The balance of viscosity of HA and elasticity of κ-ca in combination with fumed silica creates a paste-like texture ideal for Direct Ink Writing.

Combining the three components at a ratio of 1+1+1 (3 wt% Flink) forms a viscoelastic and shear thinning ink with synergistic properties (Figure 1b). 3 wt% Flink is shear thinning over the entire applied shear rate regime whereas at increasing concentrations of 6 wt% (2+2+2 ratio) and 9 wt% (3+3+3 ratio) only the overall viscosity is increased while still maintaining shear thinning. Shear thinning is crucial for Direct Ink Writing, since at an applied pressure the ink liquefies allowing for unhindered flow through the nozzle. Before and after pressure application, the ink forms a viscoelastic network ensuring shape retention of the printed object. Indeed, all three Flinks form viscoelastic networks, which are predominantly elastic at low applied strains and therefore form self-sustaining inks. Viscosity return curves (from high to low viscosities) and repeated oscillatory measurements reveal that the ink properties (elasticity and viscosity) are not lost during filling of the material into syringes and repeated mixing steps, simplifying practical handling. Elastic and viscous forces are therefore high enough to prevent sedimentation and phase separation of the composition while printing and even when stored over days.

To simulate the mechanical forces acting on the ink during Direct Ink Writing and after deposition, high (70 s⁻¹) and low (0.1 s⁻¹) shear rate cycles were applied (Figure 1c). All the presented inks show an immediate recovery in viscosity even after multiple cycles. To simulate the elastic recovery after deposition, an oscillatory time sweep at constant amplitude and frequency was interrupted by a shear step of 70 s⁻¹. The elastic modulus recovery was instantaneous for all ink designs, sufficient for structure retention during 3D printing.

After extrusion a sufficient yield stress is critical to ensure structure retention of the print. As expected, increasing Flink concentrations also show higher yield stresses (Figure 1d). The 1 wt% Flink has a yield stress and a shear thinning behaviour, however the elastic modulus under rest (Figure 1b) is not high enough to enable structure retention after deposition. However, Flinks above 1.5 wt% (1.5+1.5+1.5 ratio, total combined component concentration: 4,5%) provide ideal rheological properties for 3D printing while still allowing the entrapped bacteria to move around freely. The sustaining properties of the hydrogel (elastic and viscous recovery) recover fast enough to avoid distortion during 3D printing. Therefore, equal component distribution compostions having above 4.5 wt% total component concentration were chosen as 3D printing inks for the inclusion of bacteria.

### Example 2: modified, photo-curable glycidyl methacrylate HA (GMHA)

Using multi-material DIW of functional living inks, bacteria can be incorporated and grown in specific regions of the printed structures with high accuracy and freedom of shape. The mechanical properties of the Flink can be further tuned to obtain stronger self-supporting structures by the in-situ production of a cellulose network or by replacing HA with a modified, photo-curable glycidyl methacrylate HA (GMHA). With the rheologically optimized Flink at 6 wt% (2% glycosaminoglycan, 2% polysaccharide and 2% FS), shapes of any kind can be printed at high accuracy and small length scales (Figure 2a-c). Using the multi-material 3D printing approach, *B. subtilis* (stained with Nile Red) and *P. putida* (stained with DAPI) were imprinted into specific regions illustrating that different bacteria can be incorporated into separate regions (Figure 2d). Therefore, incompatible types of bacteria and bacteria with different nutrient requirements can be combined within the same structure. With a spatial separation, sequential and competitive processes occurring in biofilms can be studied in three dimensional scaffolds. By using glycidyl methacrylate HA (GMHA), the hydrogel can now be photo-cured and rendered water insoluble (Figure 2e). The polymerization reaction is immediate and non-toxic as it is performed at a low exposure dose and harmless wavelength (90 mW, 365 nm, 60 s). The cross-linked hydrogel swells to 1.5 times its size when immersed in the same bacterial medium. In contrast, the grid swells to double its original size when submerged in water. Induced swelling allows programming of a shape change and thus controlling the permeability of the cross-linked network by altering the mesh size. Adaptable network sizes are important to control bacterial release, uptake of nutrients and hence to create a stimuli-responsive, living material. To compare the network properties of the Flink after polymerization and after bacterial growth, amplitude sweeps were performed. After photo-polymerization of GMHA an increase in elastic (G') and viscous (G") properties can be observed in comparison to the not polymerized hydrogel (Figure 2f). The increase of G" with increasing strain is an indication of an interconnected network formed through polymerization, as it displays a weak strain overshoot. The printed structure is self-sustaining after polymerization and can be lifted up as an interconnected structure. A reinforced hydrogel is also formed by exploiting the biofilm formation of *A. xylinum,* a cellulose forming bacteria (Figure 2g). Cellulose formation in the Flink strengthen the hydrogel, as observed by an increase in G' and G". Further strengthening can be induced by UV polymerization after cellulose growth (Figure 2h). With this technique, a stronger hydrogel is formed with sufficient mechanical strength to be handled and swelled in various media.

### Example 3: 3-dimensional scaffolds capable of phenol degradation and in situ cellulose formation

Complex multi-functional living scaffolds capable of phenol degradation and *in situ* bacterial cellulose formation are created by 3D printing of hydrogels containing *P. putida* and *A. xylinum.* Immobilization of *P. putida* leads to a structure capable of degrading phenol by converting it into biomass. In-situ cellulose formation by *A. xylinum* leads to fully biocompatible hydrogels with potential biomedical applications. Bacterial cellulose formation can be controlled by oxygen availability and viscosity of the hydrogel ink.

In biotechnological applications bacteria are often immobilized in hydrogels such as agar or alginate to increase the product yield, protect the bacteria from environmental influences, and to permit growth. Here we demonstrate that bacteria can be immobilized and grow in a hydrogel formed by 3D printing. As a proof of concept, we 3D printed *P. putida* DSM 50222, a phenol degrading strain, in a cross-linked Flink and observed the degradation of phenol over time was followed by UV adsorption (Figure 3 a). First, 'free bacteria' and 'immobilized bacteria' were incubated in a minimal salts medium with phenol as the only available carbon source. Grid-immobilized bacteria (with 1% bacterial cell migration) showed a slow growth with a complete degradation after 120 hours, whereas free-floating bacteria, at the same concentration, showed complete degradation after 40 hours. Not surprisingly, the phenol degradation with the gel-enclosed bacteria was slower than the free-floating control, as the migration of bacteria out of the gel structure was measured as 1 %. Therefore, the free bacteria concentration in the medium around the grid was 100 times less than in the control. This led to a slower total degradation with immobilized bacteria than in the free-floating media. However, it was observed that phenol is degraded in the grid itself, as final OD values, and therefore bacteria concentration, showed a lower growth of free floating bacteria in comparison to the control after complete phenol degradation. Therefore, phenol is not only degraded by free-floating bacteria, but also by the bacteria present inside and on the surface of the grid structure. After phenol was fully degraded, bacterial growth ceased. A second incubation of the grid, after washing it twice in minimal media, led to a much faster degradation of phenol within 30 hours and a rapid bacterial increase to maximum numbers (Figure 3b). The degradation time after the second incubation step is even faster when compared to the free-floating control, due to a higher concentration of bacteria now present in the grid. Staining of the grid with ethidium bromide shows the presence of DNA across the whole grid, indicating that growth took place inside of the gel, whereas the control grid is completely transparent (Figure 3c). Hence, bacterial growth and metabolic activity was secured through immobilization in the printed structure. This artificial immobilization, mimicking biofilms as natural habitat, allows the control of bacterial metabolisms in predesigned environments.

The inventors suggest that biotechnological applications can be improved by exploiting a hierarchical architecture creating a greater total surface accessible by Direct Ink Writing (DIW). Following the scare-cube law, often seen in nature, the contact area between bacteria and media is thereby enlarged. Larger interfaces allow for an efficient exchange of nutrients and metabolic products as known from the capillaries in the circulatory system. Furthermore, the composition of the hydrogel can be locally adapted to meet the requirements for an optimal metabolic output of the enclosed bacteria strains. Last, the local adjustment of the mesh-density of the hydrogel results in a controlled release of bacteria, requisite for the process. Therefore, additive manufacturing of bacteria-enclosed scaffolds might potentially be used to optimize their metabolic activity by firstly exploiting the total available surface through the flexibility in shape and secondly, by locally tuning the hydrogel composition to match the requirements for highest bacteria activity. Hence, biotechnological applications of whom spatial and time-dependent release are requisite, can conceivably benefit from multi-material additive manufacturing.

Another example of added functionality through bacterial incorporation is demonstrated by the bacterium *A. xylinum.* When *A. xylinum* is embedded in the hydrogel and incubated for 4-7 days, bacterial cellulose is formed in the 3D printed scaffold, as visualized by staining the grown cellulose with Fluorescent Brightener 28, a cellulose specific fluorescent dye and by SEM (Figure 3d and e). After 3D printing and cellulose growth, most of the ink components can be washed out only leaving a network of nano-fibrillated bacterial cellulose. To analyze *A. xylinum* growth as a function of oxygen and viscosity, circular prints of 3 wt%, 4.5 wt% and 6 wt% (total of glycosaminoglycan, polysaccharide and FS components) Flink were covered with two glass slides so that air was only accessible from the side (Figure 3f). Bacteria only produced cellulose directly at the rim of the printed structure, because the proliferation of *A. xylinum* and therewith the production of bacterial cellulose highly depends on oxygen. Similarly, oxygen availability also limits the pervasion depth of bacteria inside the 3D printed structure. However, with an appropriate geometry and a corresponding ink formulation, the oxygen dependency of *A. xylinum* can be fulfilled for example in combination with oxygen producing cyanobacteria.

The growth of cellulose is furthermore a function of viscosity, as growth of cellulose is directly related to *A. xylinums* bacterial movement. At low viscosities (3 wt% Flink) cellulose formation is high, visible by the bright ring of densely cross-linked nano-fibrillated cellulose network (Figure 3f). With increasing viscosity, less bacterial cellulose growth was observed at the rim of the print. At higher Flink concentrations only little biofilm was formed as a result of immobilizing the bacteria inside the gel and restricting them in their mobility. However, at a concentration of 4.5 wt% Flink, a highly interconnected bacterial cellulose network was formed while maintaining a high enough elasticity and yield stress to ensure printing accuracy (see Figure 3).

To illustrate the full potential of *A. xylinum* embedded in the Flink, a custom-built four axis 3D printer capable of scanning and printing on an arbitrary, non-planar surface was used to deposit the bacteria containing hydrogel onto a substrate representing a human face. After incubating the face under optimal growth conditions (30°C, high humidity, 4 days), the bacterial cellulose was visualized using the fluorescent dye. In the region of the forehead, less cellulose formation was visible due to a thinner hydrogel thickness compared to the thickness around the eyes, nose and mouth. Based on the emerging importance of bacterial cellulose as skin replacements or as tissue envelops in organ transplantation, the possibility to form bacterial cellulose in any desired 3D shape allows to apply bacterial cellulose directly onto the site of interest without risking skin detachment upon movement. For example, regions that are under stronger mechanical tensions, e.g. the elbow, can therefore be reinforced directly with the growth of a cellulose scaffold. Long-term medical applications will benefit from the presented multi-material 3D printing process by locally exerting bacteria where needed.

The present specification provides a 3D bacteria printing platform which allows to produce multifunctional living materials. By using this additive manufacturing approach, the inventors were able to localize bacteria at desired locations in arbitrary shapes to form biomaterials with a living and adaptable property. This enables applications in the field of biotechnology, biomedical engineering, and biomaterials.

In the field of biotechnology, the spatial and temporal control of bacteria inside materials allow forming customized immobilization matrices tuned to match the requirements of the fermentation process. For instance, by exploiting the multimaterial approach, bacteria can be incorporated in heterogeneous materials with substructures designed for growth and for mechanical stability. Through the freedom of shape provided by 3D printing, bacteria could therefore be brought into a custom-made shape for diverse applications such as in biofiltration and biosensors. Furthermore, the freedom of shape provided by 3D printing facilitates modifying the rate of release of bacteria from the material, as for example the release of drugs is dependent on their shape. In most classical biotechnological applications, only one type of bacteria is immobilized. However, as shown by previous studies, co-cultures, as typically found in nature, exhibit interesting effects such as combined growth to form photosynthetic living matter and mixed cultures for bioenergy production. Both applications would greatly profit from this platform, as now a shape can be created for living matter and co-cultures can be grown in separate regions while still profiting from the vicinity to each other.

The invention can also be applied advantageously in the biomedical immobilization of bacteria. So far, bacteria on biomedical products were prevented to avoid severe infections. However, for areas with a large bacteria concentration such as mucosal areas and skin, functional bacteria such as probiotic bacteria could be embedded together with the transplanted tissue, providing a natural and effective barrier against pathogens. Future applications could therefore arise in the biomedical field where bacteria are imprinted purposely on skin or gut improving and re-establishing our normal healthy flora.

### Measurement protocol:

The rheological properties were measured on a strain and stress controlled rheometer. The viscosity was obtained in strain rate controlled measurements at shear rates from 0.01 to 100 s⁻¹ in time-controlled measurements. To measure the elastic (G') and viscous (G") properties, oscillatory amplitude sweeps were carried out at a frequency of 1 Hz with deformations ranging from 0.01 to 100 %. The yield stress values correspond to the dynamic cross-over point between G' and G".

To study the recovery behavior of the ink after printing, the shear forces exerted during extrusion were simulated by shearing the ink at a shear rate of 70 s⁻¹ for 100 s, followed by the measurement of the viscosity over time at a shear rate of 0.1 s⁻¹. Additionally, the inks wered measured in an oscillatory time sweep, followed by a shear period of 1 min at 70 s⁻¹ and finally by a time sweep at varying amplitude and frequencies to obtain the elastic recovery of the ink.

Whererever reference is made to shear forces, yield stress or elastic (G') and viscous (G") properties herein, the referenced values are deemed to be determined by the above protocol.

## Claims

1. A method for providing a three-dimensional structure comprising viable cells, comprising the steps of
a) providing an aqueous composition comprising
i) a glycosaminoglycan component,
ii) a gel forming polysaccharide component,
iii) a thixotropic agent component,
iv) a growth medium component,
v) a population of live cells,
wherein the sum of the mass of each the glycosaminoglycan component, the gel forming polysaccharide component and the fumed silica component, in relation to the total mass of the aqueous composition, ranges from 3% to 9% (w/w).
b) repeatedly exerting a shear force on a volume of said aqueous composition, and
c) applying said volume of said aqueous composition to a three-dimensional structure.

2. The method according to claim 1, wherein
a. said gel forming polysaccharide component is a carrageenan or agar and/or
b. the thixotropic agent component is selected from the group comprising fumed (pyrogenic) silica and smectic clay minerals.

3. The method according to any one of the preceding claims, wherein the sum of the mass of each the glycosaminoglycan component, the gel forming polysaccharide component and the fumed silica component, in relation to the total mass of the aqueous composition, ranges from 4,5% to 9% (w/w).

4. The method according to any one of the preceding claims, wherein each of the mass of the glycosaminoglycan component, the mass of the gel-forming polysaccharide component and the mass of the thixotropic agent component, in relation to the total mass of the aqueous composition, are individually and independently from each other selected to range from 1,0 % to 5,0% (w/w).

5. The method according to any one of the preceding claims, wherein the mass of the glycosaminoglycan component, the mass of the gel-forming polysaccharide component and the mass of the thixotropic agent component are selected so that the component having the highest mass among said components is equal or less (≤) than 1,5 times, particularly ≤ 1,2 times, the lowest mass among said components.

6. The method according to any one of the preceding claims, wherein the sum of the mass of the glycosaminoglycan component and the mass of the gel-forming polysaccharide component is selected to range between 1 1/3 times and 3 times the mass of the thixotropic agent component.

7. The method according to any one of the preceding claims, wherein the mass of the glycosaminoglycan component, the mass of the gel-forming polysaccharide component and the mass of the thixotropic agent component are selected from Table 1.

8. The method according to any one of the preceding claims, wherein the shear stress applied in step b) is 100 Pa or greater.

9. The method according to any one of the preceding claims, wherein said glycosaminoglycan component comprises photopolymerizable carbon-carbon double bonds, and wherein the three-dimensional structure is exposed to light suitable for polymerization of said photopolymerizable carbon-carbon double bonds.

10. The method according to any one of the preceding claims, wherein said population of live bacterial cells comprises, or essentially consists of, cells that deposit a solid matrix selected from cellulose, calcium carbonate and magnetite.

11. The method according to claim 10, wherein said cells are selected from
a. Acetinobacter xylinum, Gluconacetobacter hansenii, Gluconacetobacter xylinus, Acetobacter sp., Sporosarcina pasteurii, Deleya halophile and Proteus mirabilis, particularly selected from the group comprising A. xylinum strains BRC5, BPR2001, E25 or NUST4.1, G. hansenii strain PJK, Acetobacter sp. strains V6 or A9, G. xylinus strains K3, IFO 13773 and RKY5, Sporosarcina pasteurii ATCC 11859, or from
b. the group comprising Rhizobium, Alcaligenes, Azotobacter, Pseudomonas, Salmonella, Sarcina ventriculi and / or
c. magnetotactic bacteria, particularly selected from the group comprising *Magnetospirillum gryphiswaldense* MSR-1, *Magnetospirillum magneticum* AMB-1, *Magnetospirillum magneticum* MGT-1, *Magnetovibrio* MV-1, *Magnetococcus* sp. MC-1, *Magnetospirillum* sp. WM-1 *Magnetospirillum magnetotacticum* MS-1 and *Desulfovibrio magneticus* RS-1.

12. An aqueous composition comprising
i) a glycosaminoglycan component, particularly a hyaluronic acid component,
ii) a gel forming polysaccharide component,
iii) a thixotropic agent component, particularly a fumed (pyrogenic) silica component,
iv) a growth medium component, and
v) a population of live bacterial cells,
wherein the sum of the mass of each the glycosaminoglycan component, the k-carageenan component and the fumed silica component, in relation to the total mass of the aqueous composition, ranges from 3% to 9% (w/w).

13. A solid gel volume having a discrete three-dimensional form, said solid gel comprising
vi) a glycosaminoglycan component, particularly a hyaluronic acid component,
vii) a gel forming polysaccharide component, particularly a k-carageenan component,
viii) a thixotropic agent component, particularly a fumed (pyrogenic) silica component,
ix) a population of live bacterial cells, and
x) optionally, a growth medium component,
wherein said solid gel is **characterized by**
- an elastic modulus of 0.1-10 kPa and/or
- a yield strain of 10-50% and/or
- a dynamic yield stress of 10-500 Pa and/ or
- an instant recovery (less than a second) of the viscoelastic network.

14. The solid gel volume according to claim 13, wherein the mass of the glycosaminoglycan component, the mass of the gel-forming polysaccharide component and the mass of the thixotropic agent component are selected from Table 1.

15. Use of a solid gel volume according to claim 13, wherein the bacterial cells are P. putida cells, in a method for degrading phenols.
